# EUROPEAN PATENT APPLICATION

(11) **EP 3 760 120 A1**
(43) Date of publication of application: **06.01.2021**
(21) Application number: 19184467.9
(22) Date of filing: 04.07.2019
(51) Int. Cl.: A61B 5/11, A61B 5/00, A61B 5/103

(54) **WEARABLE CUEING SYSTEMS FOR ASSISTING A SUBJECT WITH WALKING**

(71) Applicant: Koninklijke Philips N.V., 5656 AG Eindhoven (NL)
(72) Inventor: VALENTI, Giulio, 5656 AE Eindhoven (NL); BALDUS, Josef Heribert, 5656 AE Eindhoven (NL)
(74) Representative: de Haan, Poul Erik

(57) **Abstract**

A system and method for providing cues to assist a subject or patient's walking. The system obtains sensory information responsive to movement of the subject, which is processed to detect a non-zero movement of the subject. In response to detection of a non-zero movement, a timing of a cue is defined or controlled by a cueing module.

## Description

### FIELD OF THE INVENTION

The present invention relates to the field of cueing systems, and in particular to cueing systems for assisting a subject with walking.

### BACKGROUND OF THE INVENTION

It is common amongst patients who live with a neurological or neural disorders, such as Parkinson's disease, to experience "freezing". This typically manifests itself as the patient feeling that they are unable to move or perform a certain action. In particular, it is known for freezing to affect walking, causing a walking process or motion of the patient to be interrupted. It is clear that freezing has a significant impact on the day-today lives of such patients.

It has been observed that cueing systems, which provide visual/audio cues for the subject, help overcome a freezing event. Typical cueing systems provide cues in response to detection of a freezing event occurring, i.e. when a subject stops moving, although it is also known for cueing systems to continually provide cues to a subject when activated, i.e. without detecting the occurrence of a freezing event.

There is an ongoing desire to improve the adaptability and efficacy of cueing systems.

### SUMMARY OF THE INVENTION

The invention is defined by the claims.

According to examples in accordance with an aspect of the invention, there is provided a wearable cueing system for providing user-perceptible cues for encouraging a walking process of a subject.

The wearable cueing system comprises: a cueing module adapted to generate user-perceptible cues; a sensor adapted to generate sensory information responsive to movement of the subject; a cueing control module adapted to process the sensory information to detect a non-zero movement of the subject; and control the cueing module to control the timing of a cue in response to the detected non-zero movement of the subject.

Rather than attempting to identify a freezing event, the present invention proposes to adjust cues based on detection of non-zero movement of the subject. In particular, the cueing system detects occasions when a subject is moving and adjusts or controls a timing of a cue (i.e. when the cue is provided) based on detecting such an occasion. Adjusting a timing of a cue may comprise timing a switch from a first cue to a second, different cue, or may comprise generating a new cue at a particular point in time.

This may, for example, allow a cue for a next step of the subject to be generated in response to detecting a current step of the subject successfully taking place or to take place at a predicted time of a next step of the subject. This allows the cueing system to respond to successful movement of the subject and adjust a cueing accordingly. Embodiments also envisage automatic starting of a cueing system, e.g. in response to a user beginning to walk.

The proposed cueing system is therefore more adaptable and responsive to movement of the subject.

In some embodiments, the cueing control module is adapted to process the sensory information to detect a non-zero movement indicating a change in gait phase of the subject; and control the cueing module to control the timing of a cue based on the detected change in gait phase.

A gait cycle of the subject can be divided into a number of gait phases. Conventionally, a gait cycle is divided into a "swing" and a "stance" phase, the swing phase indicating an airborne movement of the foot and a stance phase indicating a largely stationary foot. By changing or generating a cue based on detecting a change in the gait phase (i.e. detecting a transition between two gait phases), a successful completion or initiation of a step by the subject, i.e. successful avoidance of freezing, can be detected. The cue can then be generated/updated to guide the next step of the subject.

It is envisaged that a gait cycle can be divided (or sub-divided) into other phases, which are also contemplated as falling with the meaning of the term "phase". For example, a stance phase can be sub-divided into five separate movements or phases, namely: initial contact, loading response, mid stance, terminal stance and toe off.

In embodiments, the cueing control module is adapted to process the sensory information to determine a time at which a particular movement of the subject happens (e.g. when the subject takes a step or begins to move). Generation or modification of a cue is performed in response to the determined time.

In particular, the cueing control module may be adapted to detect successful avoidance of freezing during a walking motion of the subject, by analyzing the sensory information, and modify or generate a cue in response to this detection.

In some embodiments, the cueing control module is adapted to process the sensory information to detect the subject changing from a stance phase to a swing phase of a gait cycle; and controlling the cueing module to control the timing of a cue based on the detected change from the stance phase to the swing phase.

The sensor may be adapted to generate sensory information responsive to a movement of the subject's foot; and the cueing control module may be adapted to process the sensory information to detect a movement of the subject's foot and control the cueing module to control the timing of a cue based on the detected movement of the subject's foot.

Detecting movement of the patient's foot enables successful initiation or completion of a step to be readily detected. It also improves an accuracy in detecting an appropriate walking movement of the subject.

In embodiments, the cueing control module is adapted to process the sensory information to detect a lifting of the foot from a ground surface and control the cueing module to control the timing of a cue in response to detecting a lifting of the foot.

The cueing control module may be adapted to process the sensory information to detect a lifting of the subject's heel from a ground surface and control the cueing module to control the timing of the cue in response to detecting a lifting of the subject's heel.

In at least one embodiment, the cueing control module is further adapted to: process the sensory information to detect the subject beginning a walking process; and control the cueing module to generate a cue in response to detecting the subject beginning the walking process. In other words, the cueing control module can initiate a cueing process or procedure in response to detecting movement of the subject.

The sensor may comprise an accelerometer or a gyrometer. In other embodiments, the sensor may comprise a microphone adapted to monitor a sound of the subject's movement. In yet other embodiments, the sensor may comprise a pressure sensor for sensing a pressure of the user's foot against a ground surface, which is indicative of movement.

Preferably, the cueing module comprises at least one of the following: a laser, a projector, a speaker, a haptic output element and a display. Thus, the cueing module may comprise any audio, visual or haptic feedback system capable of providing a user-perceptible cue to the subject. The cueing module thereby effectively acts as a user interface.

In some embodiments, the cueing module may be adapted to be incorporated into a pendant to be worn around the subject's neck. In some embodiments, the cueing module may be adapted to be mounted in or on footwear of the subject, e.g. a laser mounted in a shoe. More generally, the wearable cueing system may be adapted to be wearable around the neck and/or the wrist of the subject.

According to other examples in accordance with an aspect of the invention, there is provided a method of operating a wearable cueing system for providing user-perceptible cues for encouraging a walking process of a subject, the wearable cueing system comprising a cueing module adapted to generate user-perceptible cues and a sensor adapted to generate sensory information responsive to movement of the subject; providing dynamic gait-aware cues to a subject.

The method comprises: obtaining, from the sensor, sensory information responsive to movement of the subject; processing the sensory information to detect a non-zero movement of the subject; and controlling the cueing module to control the timing of a cue based on the detected non-zero movement of the subject.

In some embodiments, the method is adapted wherein: the step of processing the sensory information comprises processing the sensory information to detect a non-zero movement indicating a change in gait phase of the subject; and the step of controlling the cueing module comprises control the cueing module to control the timing of a cue based on the detected change in gait phase.

In at least one embodiment, the method is adapted wherein: the step of obtaining comprises obtaining sensory information responsive to a movement of the subject's foot; and the step of processing the sensory information comprises processing the sensory information to detect a movement of the subject's foot; and the step of controlling the cueing module comprises controlling the cueing module to modify the cue based on the detected movement of the subject's foot.

Optionally, the step of processing the sensory information comprises processing the sensory information to detect a lifting of the subject's heel from a ground surface; and the step of controlling the cueing module comprises controlling the cueing module to modify the cue in response to detecting a lifting of the subject's heel.

According to other examples in accordance with an aspect of the invention, there is provided a computer program comprising code means for implementing any herein described method when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a cueing system according to an embodiment of the invention;
Figure 2 illustrates a method of controlling a cueing system according to an embodiment of the invention; and
Figure 3 illustrates a cueing system worn by a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for providing cues to assist a subject or patient's walking. The system obtains sensory information responsive to movement of the subject, which is processed to detect a non-zero movement of the subject. In response to detection of a non-zero movement, the timing of a cue is defined or controlled by a cueing module.

Embodiments are at least partly based on the realization that a more adaptive cueing system and methodology would help a subject living with Parkinson's with their gait and/or walking. In particular, it has been recognized that generating/modifying a cue based on movement of the subject enables the cueing system to adapt to successful movement (i.e. non-freezing) of the subject, thereby enabling an iterative cueing system to be provided.

Illustrative embodiments may, for example, be employed in wearable cueing systems for cueing a subject, e.g. living with Parkinson's disease or similar conditions, to aid and encourage a walking process.

In the context of the present application, the term "subject" is used interchangeably with the term "patient", and refers to a user of the cueing system.

In the present application, wearable means that it can be carried or mounted by the user, e.g. in the form of a mobile/cellular phone or as dedicated wearable hardware.

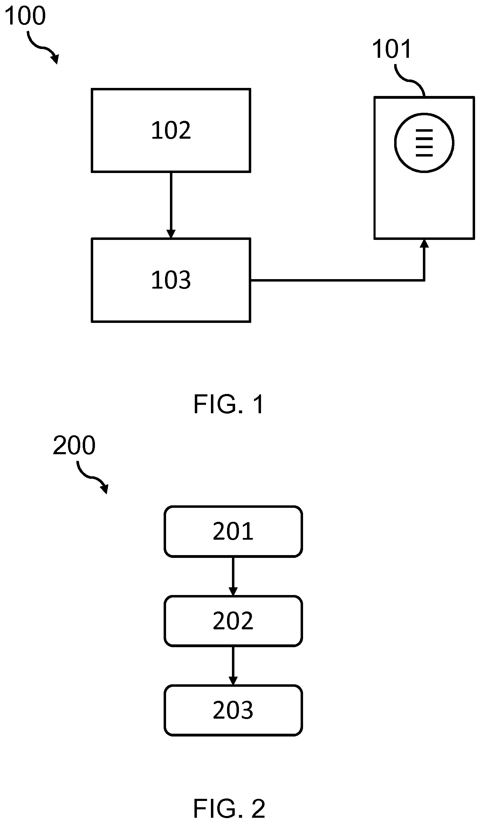

Figure 1 illustrates a cueing system 100 according to an embodiment of the invention.

The cueing system 100 is designed to be wearable by a subject (e.g. to hang around their neck or be mounted upon the wrist, although other methods are contemplated such as being integrated into mobile/cellular phones). Preferably, the cueing system 100 comprises dedicated hardware for being directly worn by the user, rather than being integrated into existing hardware, such as mobile/cellular phones.

The cueing system 100 comprises a cueing module 101 adapted to generate cues for encouraging or prompting a subject to perform or continue a walking process.

The cueing module 101 may comprise, as illustrated, a speaker for generating an audible cue. In other embodiments, the cueing module 101 comprises a visual output module for generating a visual cue. Suitable examples of a visual output module include a laser, a display and a projector system. It yet other embodiments, the cueing module 101 comprises a haptic output element, such as a vibrating element (e.g. integrated into a wrist-mountable device).

Thus, the cueing module 101 may be adapted to generate an audio output, a visual output and/or a haptic output, which acts as a cue for prompting the subject to perform or continue a walking process. Thus, a cue is a user-perceptible (e.g. audio/visual/haptic) output. Suitable cues will be readily apparent to the skilled person and are known in the prior art.

The cueing system 100 further comprises a sensor 102 adapted to generate sensory information responsive to movement of the subject. The sensory information may, for example, be carried by at least one sensory signal (not shown) generated by the sensor 102.

The sensor 102 preferably comprises an accelerometer. An accelerometer is particularly sensitive to movement, thereby allowing suitable sensory information to be generated (which sensory information may be labelled "accelerometer data"). In other examples, the sensor 102 may comprise a gyroscope (sometimes labelled a gyrometer), which is responsive to changes in orientation or angular velocity such as those generated during a walking motion, which outputs gyrometer data that can be processed in a similar manner to accelerometer data.

In yet another example, the sensor 102 may comprise a microphone adapted to record a sound of the subject's movements and generate sensory information accordingly ("microphone data"). It will be appreciated that movement of the subject, e.g. when walking, generates sounds which could be detected by a microphone for subsequent processing (e.g. to identify the initiation/end or an ongoing occurrence of a phase of the walking process).

As another example, the sensor 102 may comprise a pressure sensor adapted to sense a pressure of the subject's foot on a ground surface and generate sensory information accordingly (which sensory information may be labelled "pressure data"). It will be appreciated that as a subject's foot contacts or is raised from a ground surface, the pressure applied by the subject's foot to the ground surface will change. Thus, a pressure sensor can indicate non-zero movement of the subject's foot, and thereby the subject.

The skilled person will readily appreciate alternative methods of generating sensory information responsive to movement of the subject, any of which could be employed in the present invention.

The cueing system 100 further comprises a cueing control module 103. The cueing control module 103 is adapted to control the timing of cues generated/output by the cueing module 101.

In particular, the cueing control module 103 is adapted to receive and process the sensory information to identify or detect a non-zero movement of the subject. The cueing control module then adapts the point in time at which the cue is generated or output by the cueing module 101 based on the detected non-zero movement.

The sensory signal(s) that may be generated by the sensor 102 may be passed to the cueing control module 103 over a wire or wireless transmission medium, e.g. BlueTooth or WiFi, as would be well known to the skilled person.

To control the cueing module, the cueing control module 103 may generate one or more control signals for the cueing module 101 to receive. The control signal(s) may be passed to the cueing module over a wire or wireless transmission medium, e.g. BlueTooth or WiFi, as would be well known to the skilled person.

The cueing control module 103 may be adapted to detect movement of the subject indicating of initiation of a walking process. The cueing control module may then control the cueing module to generate a series of cues to encourage or support the walking process of the subject. This effectively provides a cueing system that automatically begins cueing the subject upon detection of the subject beginning to move. In other words, the timing of the cues is responsive to a detected movement of the subject.

In preferred examples, the cueing control module is adapted to respond to movement of the subject during a walking process. This may be in addition or in place of responding to initiation of a walking process. In particular, the cueing control module may identify a time at which a (certain) movement occurs, and control when the cue occurs based on the detected time of the movement.

The cueing control module 103 may be adapted to trigger a (certain) cue in response to detecting (a certain) movement. In other examples, the cueing control module 103 is adapted to change from a first cue to a second, different cue in response to detecting (a certain) movement.

Preferably, the cueing control module is adapted to process the sensory information to identify a predetermined movement, such as a movement indicating an ongoing gait phase or a change of gait phase. The cueing control module 103 may, for example, detect the subject changing from a stance phase to a swing phase of a gait cycle, i.e. detect a transition between two gait phases. This may, for example, be performed by determining a time at which a swing phase begins. The cueing control module 103 may then control the cueing module to control a timing of a subsequent cue based on the detected change from the stance phase to the swing phase. Thus, the timing of the cue can be updated for each successful step, or avoidance of freezing, of the subject.

In other words, the cueing control module may be adapted to detect successful avoidance of freezing during a walking motion of the subject, by analyzing the sensory information, and modify or define a timing of the cue in response to this detection. This enables the cueing system 100 to respond and adapt to the subject successfully avoiding freezing, providing a more adaptive system.

In one example, the sensor 102 may comprise an accelerometer and the cueing control module 103 may be adapted to process the accelerometer data (the sensory information) to detect the moment or instant that a heel of the subject's foot is lifted off the ground - indicating successful avoidance of freezing. This may be indicated in the accelerometer data as a strong upward and/or forward acceleration. The cueing control module 103 may then control the cueing module 101 responsive to detecting the lifting of the heel by switching the cue output by the cueing module from a first cue to a second different cue. In this way, the timing of the switch of the cue is responsive to a detected (particular) movement of the subject. The second, different cue may be a cue suitable for a next step of the subject (e.g. identifying a foot fall location for the subject). Thus, as the subject steps forward, the cue provided to the subject changes to provide an adaptive and self-updating system that responds to successful movement of the subject.

A subject's gait cycle can be divided into a number of phases (which can be labelled "gait phases"). Conventionally, a gait cycle is divided into a "swing" and a "stance" phase, the swing phase indicating an airborne movement of the foot and a stance phase indicating the foot being in contact with a ground surface. By changing or generating a cue based on detecting a change in the gait phase (i.e. detecting a transition between two gait phases), a successful completion or initiation of a step by the subject, i.e. successful avoidance of freezing, can be detected. The cue can then be generated/updated to guide the next step of the subject.

It is envisaged that a gait cycle can be divided (or sub-divided) into other phases, which are also contemplated as falling with the meaning of the term "phase". For example, a stance phase can be sub-divided into five separate movements or phases, namely: initial contact, loading response, mid stance, terminal stance and toe off. As another example, a swing phase can be sub-divided into at least three separate movements or phases, namely: initial swing, mid swing and terminal swing.

It is appreciated that other methods of divided a gait cycle into gait phases may be used to advantage, and may depend upon the clinical context or jurisdictional differences or standards.

The cueing control module 103 may be adapted to process the sensory information generated by the sensor 102 to detect a transition into one or more of these gait phases and respond to a change in the gait phase, e.g. by appropriately timing a subsequent cue or cues generated by the cueing module.

Thus, detection of entry into a new gait phase by the cueing control module 103 can be used to control when (i.e. a point in a time at which) a cue is generated by the cueing module 101.

In a first scenario, the cueing module 101 is adapted to generate audible cues for encouraging a skilled person to walk. A first cue may provide an instruction to take a left step (e.g. "Left") and a second cue may provide an instruction to take a right step (e.g. "Right"). The cueing control module may detect when a subject moves from a swing phase to a stance phase, and switch between the two cues in response to this detection. Thus, as a subject completes a step, they are automatically instructed to take a next step. This helps encourage the subject to perform the walking process.

In a second scenario, the cueing module 101 is adapted to generate a visual cue (e.g. a projected cue) for encouraging a skilled person to walk. A first cue may provide an instruction to aid the user in taking a left step (e.g. "Lift your left leg as if walking through treacle" or a diagram illustrating the same) and a second cue may provide an instruction to aid the user in taking a right step (e.g. "Lift your right leg as if walking through treacle" or a diagram illustrating the same). The cueing control module may detect when a subject moves from a stance phase to a swing phase (e.g. when their foot or heel lifts from a ground surface, indicating avoidance of freezing) and switch between the two cues in response to this detection. Thus, cues can automatically updated when they are no longer required, provided an automatic and adaptive cueing system 100.

For the sake of completeness, it is noted that the cueing control module may be capable of distinguishing between left and right steps, as the skilled person will appreciate that a movement of the subject differs for each type of step. The cueing control module may be adapted to provide the appropriate cue (for a left/right step) based on this determination.

To process the sensory information, the cueing control module may obtain a most recent portion of the sensory information and process the portion to detect a (change of) gait phase, which can then be used to modify/generate the cue. The most recent portion may, for example, be a past 100ms, 500ms or 1000ms of the sensory information. Preferably, the most recent portion is acquired over a time period corresponding to a most recent gait cycle of the subject, e.g. up to two seconds.

A couple of embodiments for controlling the timing of a cue have been previously described. However, there are a number of different possible strategies for controlling (the timing of) the cue by the cueing control module.

In one example, the sensory information generated by the sensor 102 may be processed to predict a time at which the subject's foot makes contact with the ground surface (foot contact), and time a cue to be output/changed at this predicted time. This can be achieved by monitoring when historical foot contacts of the subject occur (i.e. there is a gait phase change from a swing phase to a stance phase), and predicting the timing of the next foot contact of the subject. A foot contact is detectable as a strong upward deceleration (in accelerometer data) or as a sudden increase of pressure under the heel (in pressure data). The prediction for a timing of a next foot contact can be updated after each measured previous foot contact of the subject.

In another example, the sensory information generated by the sensor 102 may be processed by the cueing control module to predict a time at which the subject desires to initiate a swing of their foot, and time a cue to be output at this predicted time. Typically, a subject desires to initiate a swing of their foot at a time when both feet are in contact with a ground surface (double support). Double support is an example of a gait phase. In one embodiment, the cueing control module may be adapted to determine an average double support time from the past N steps (where N is a real, positive number, e.g. 3, 5 or 10). The cueing control module may then detect an initiation of a current double support, and time a cue to be output at the predicted end of the current double support period (e.g. after the average double support time has elapsed). A double support can be detected by detecting when pressure data indicates a pressure under both feet, or my morphological analysis of acceleration from accelerometer/microphone data.

In an alternative approach, the cueing control module is adapted to detect when a swing of the subject's foot is about to take place (e.g. by detecting when a pressure applied by the subject's foot increases, as they push of the floor to take a step). A timing for the next step can be predicted using this information, and a cue output at the predicted time. In an embodiment, if a swing is not detected (i.e. freezing occurs), then a fall back cueing strategy can be used by providing cueing at a regular tempo, having a frequency derived (e.g. equivalent to) a tempo of a past successful walk of the subject.

For improved accuracy, the cueing control module 103 may employ a machine-learning algorithm designed to detect gait phases or to predict changes of gait phase from a portion of sensory information.

A machine-learning algorithm is any self-training algorithm that processes input data in order to produce or predict output data. Here, the input data comprises sensory information responsive to movement and the output data comprises gait phases.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example portions of sensory information. The training output data entries correspond to corresponding gait phases.

Other methods of processing sensory information to identify (changes of) gait phases will be apparent to the skilled person. For example, a pattern matching algorithm may determine a similarity between obtained sensory information and different instances of reference sensory information (each instance associated with a particular gait phase or change of gait phase) and identify the most similar reference sensory information.

It is not essential for the cueing control system to obtain and process a most recent portion, and other methods of processing the sensory information may be employed. For example, the cueing control system may identify landmarks within the sensory information, e.g. where the sensory information crosses a threshold value or remains above a threshold value for a certain period of time, in order to identify a change of gait phase.

By way of example only, an upward acceleration (e.g. going above a predetermined value) may indicate that a user has lifted their foot from the floor and thereby switched from a stance phase to a swing phase. A strong downward deceleration may indicate that a user has placed their foot on the floor, and thereby switched from a swing phase to a stance phase. Other embodiments would be apparent to the skilled person.

Figure 2 illustrates a method 200 of controlling a cueing system according to an embodiment of the invention. In particular, the method 200 is adapted for operating a wearable cueing system for providing user-perceptible cues for encouraging a walking process of a subject, the wearable cueing system comprising a cueing module adapted to generate user-perceptible cues and a sensor adapted to generate sensory information responsive to movement of the subject; providing dynamic gait-aware cues to a subject.

The method 200 comprises a first step 201 of obtaining, from the sensor, sensory information responsive to movement of the subject.

The method 200 also comprises a second step 202 of processing the sensory information to detect a non-zero movement of the subject.

The method 200 also comprises a third step 203 of controlling the cueing module to control the timing of a cue based on the detected non-zero movement of the subject.

The method 200 may be appropriately adapted to perform any previously described process or embodiment, e.g. as described with reference to Figure 1.

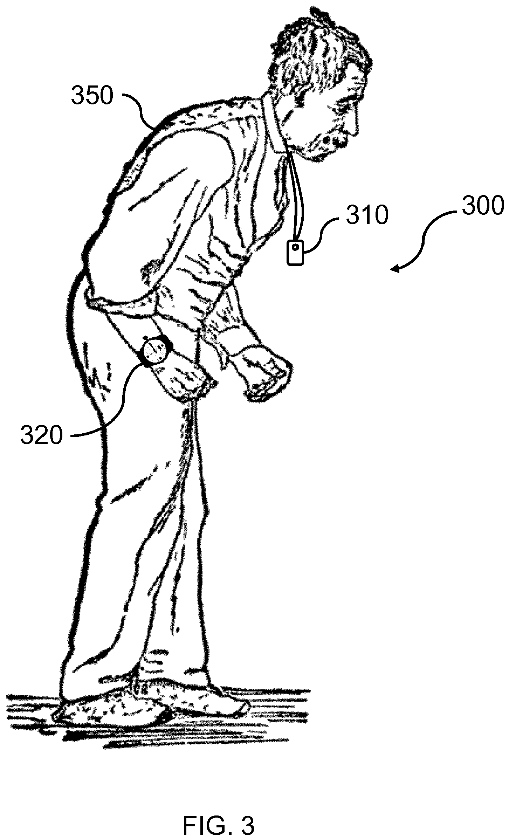

Figure 3 illustrates a wearable cueing system 300 in use, accordingly an embodiment of the invention.

The wearable cueing system 300 comprises a pendant 310 worn around a neck of the subject 350 and a wrist-mounted module 320 worn around a wrist of the subject.

The pendant 310 comprises an accelerometer (not shown) that acts as the sensor for generating sensory information responsive to movement of the subject. The wrist-mounted module 320 comprises the cueing module for generating cues, e.g. in the form of a haptic output. The cueing control module may be comprised in either the pendant 310, the wrist-mounted module 320 or another element (e.g. a mobile/cellular phone carried by the user, not shown).

The different elements of the wearable cueing system 300 may communicate with one another over any suitable wire or wireless transmission medium, e.g. BlueTooth or WiFi, as would be well known to the skilled person.

The illustrated embodiment uses a neck-worn pendant to carry the sensor, and it is recognized that neck-worn sensors are sufficiently accurate to carry out the claimed invention, whilst also being comfortable and convenient to wear. However, in other embodiments, the sensor may be mounted upon a leg (or legs) or within a sole (or soles) of the subject. Such sensors may achieve greater accuracy in detected movement of the subject, and may be more responsive to movement of the subject's foot. Sole-mounted sensors allow more flexibility in the choice of the sensor compared to other methods of wearing the sensor, e.g. allows pressure sensors to be used.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A wearable cueing system (100, 300) for providing user-perceptible cues for encouraging a walking process of a subject (350), the wearable cueing system comprising:
a cueing module (101) adapted to generate user-perceptible cues;
a sensor (102) adapted to generate sensory information responsive to movement of the subject; and
a cueing control module (103) adapted to process the sensory information to detect a non-zero movement of the subject; and control the cueing module to control the timing of a cue in response to the detected non-zero movement of the subject.

2. The wearable cueing system of claim 1, wherein the cueing control module is adapted to process the sensory information to detect a non-zero movement indicating a change in gait phase of the subject; and control the cueing module to control the timing of a cue based on the detected change in gait phase.

3. The wearable cueing system of claim 2, wherein the cueing control module is adapted to process the sensory information to detect the subject changing from a stance phase to a swing phase of a gait cycle; and controlling the cueing module to control the timing of a cue based on the detected change from the stance phase to the swing phase.

4. The wearable cueing system of any of claims 1 to 3, wherein the sensor is adapted to generate sensory information responsive to a movement of the subject's foot; and the cueing control module is adapted to process the sensory information to detect a movement of the subject's foot and control the cueing module to control the timing of a cue based on the detected movement of the subject's foot.

5. The wearable cueing system of claim 4, wherein the cueing control module is adapted to process the sensory information to detect a lifting of the foot from a ground surface and control the cueing module to control the timing of the cue in response to detecting a lifting of the foot.

6. The wearable cueing system of claim 4, wherein the cueing control module is adapted to process the sensory information to detect a lifting of the subject's heel from a ground surface and control the cueing module to control the timing of the cue in response to detecting a lifting of the subject's heel.

7. The wearable cueing system of any of claims 1 to 6, wherein the cueing control module is further adapted to:
process the sensory information to detect the subject beginning a walking process; and
control the cueing module to generate the timing of a cue in response to detecting the subject beginning the walking process.

8. The wearable cueing system of any of claims 1 to 7, wherein the sensor comprises an accelerometer.

9. The wearable cueing system of any of claims 1 to 8, wherein the cueing module comprises at least one of the following: a laser, a projector, a speaker, a haptic output element and a display.

10. The wearable cueing system of any of claims 1 to 9 adapted to be wearable around the neck or the wrist of the subject.

11. A method (200) of operating a wearable cueing system (100, 300) for providing user-perceptible cues for encouraging a walking process of a subject (350), the wearable cueing system comprising a cueing module (101) adapted to generate user-perceptible cues and a sensor (102) adapted to generate sensory information responsive to movement of the subject; providing dynamic gait-aware cues to a subject, the method comprising:
obtaining (201), from the sensor, sensory information responsive to movement of the subject;
processing (202) the sensory information to detect a non-zero movement of the subject; and
controlling (203) the cueing module to control the timing of a cue based on the detected non-zero movement of the subject.

12. The method of claim 11, wherein:
the step of processing the sensory information comprises processing the sensory information to detect a non-zero movement indicating a change in gait phase of the subject; and
the step of controlling the cueing module comprises control the cueing module to control the timing of a cue based on the detected change in gait phase.

13. The method of claim 11 or 12, wherein:
the step of obtaining comprises obtaining sensory information responsive to a movement of the subject's foot; and
the step of processing the sensory information comprises processing the sensory information to detect a movement of the subject's foot; and
the step of controlling the cueing module comprises controlling the cueing module to modify the timing of the cue based on the detected movement of the subject's foot.

14. The method of claim 13, wherein:
the step of processing the sensory information comprises processing the sensory information to detect a lifting of the subject's heel from a ground surface; and
the step of controlling the cueing module comprises controlling the cueing module to modify the timing of the cue in response to detecting a lifting of the subject's heel.

15. A computer program comprising code means for implementing the method of any one of claims 11 to 14 when said program is run on a processing system.
